Europäisches Patentamt

European Patent Office

Office européen des brevets

(11) Publication number: 0 385 153
A1

# EUROPEAN PATENT APPLICATION

(21) Application number: 90102473.7

(51) Int. Cl.5: A61M 5/50, A61M 5/315

(22) Date of filing: 08.02.90

(30) Priority: 17.02.89 IT 1947089

(43) Date of publication of application:
05.09.90 Bulletin 90/36

(84) Designated Contracting States:
AT BE CH DE DK ES FR GB GR IT LI LU NL SE

(71) Applicant: GI.BI.EFFE S.r.l.
Via Washington, 17
I-20146 Milan(IT)

(72) Inventor: Lo Duca, Carmelo
Via Val Di Sole 11
I-20141 Milan(IT)

(74) Representative: Giambrocono, Alfonso, Dr.
Ing. et al
Ing. A. Giambrocono & C. S.r.l. Via Rosolino
Pilo 19/B
I-20129 Milano(IT)

(54) Disposable syringe.

(57) In a syringe (10) for medical use, of the disposable type, the inner surface of the syringe cylindrical body (12) in which the syringe plunger (20) slides, is provided with a circular step (18) arranged in a perpendicular plane to the axis of the cylindrical body (12), the greater inner diameter portion (16) being that toward the syringe needle (24). The syringe plunger (20) is provided with a resiliency adapted to hold tightness between the plunger (20) and inner surface even if the plunger (20) is arranged in the portion (16) of the cylindrical body (12) having the larger inner diameter.

Fig. 1

EP 0 385 153 A1

## BACKGROUND OF THE INVENTION

The present invention relates to a medical use syringe and,more specifically,a disposable or single use type of syringe.

Known syringes of the above mentioned type essentially comprise a cylindrical hollow syringe body, open at one end thereof, the other end of which having a tapering shape and so designed as to receive a conventional injection needle.Within the cylindrical syringe body a syringle plunger can tight reciprocate, which can be driven by means of a plunger rod projecting from the mentioned open end of the cylindrical syringe body.

The cylindrical body and plunger rod of these known syringes are made of a substantially rigid plastic material, the syringe plunger being made of rubber or a resilient plastic material of given hardness.These syringes are usually operated in the following known way.By gripping by the fingers the free end portion of the plunger rod, the syringe plunger is moved away from its needle adjoining position,or end position,in which the plunger is usually arranged as the syringe is supplied.Thus,inside the syringe a negative pressure is generated which allows a set amount of solution drug to be withdrawn from a vial or bottle.Then the withdrawn drug is injected in a conventional well known way.

The plastic material syringes,of comparatively small cost are usually used for a single injection and then they are disposed away.This is due to obvious hygienic requirements,which at present are very stringent,mainly because of the AIDS (Acquired Immunodeficiency Syndrome) which is easily transmitted by used syringes.

Notwithstanding this,used syringes are frequently reused,mainly by drugged persons,with deleterious consequences.

Because of this reason,so-called disposable or single use syringes have been recently designed,which comprise device for preventing these syringes from being re-used.

In the Italian Patent Application N° 9,354 A/87 there is disclosed a syringe of this type.In particular, an embodiment of this syringe comprises,inside the cylindrical body in which the syringe piston slides,a circular rib lying in a plane perpendicular to the axis of the cylindrical body.Said rib has a saw tooth shape cross-section.

This saw tooth is provided with a side,that is the side facing the syringe needle,perpendicular to the inner surface of the cylindrical body,whereas the other side thereof is so slanted as to allow the plunger to be fully pushed, by its plunger rod,toward the needle, by overcoming said rib, so as to fully inject the injectable drug solution held in the syringe.

Owing to the above disclosed shape of the rib, the syringe plunger will be hindered in its return movement,if one would attempt to perform another injection of the injectbale solution.In fact,in this case,the plunger will abut against the rib face which is perpendicular to the inner surface of the cylindrical body.However,because of the resiliency of the plunger as a comparatively high pulling force is applied to the plunger rod,the plunger would be able of overcoming the mentioned rib.

In order to prevent this from occurring,between the plunger and plunger rod there has been provided a coupling arrangement which will fail as the pulling force on the plunger rod reaches a set value,which value must be less than the pulling force which is necessary to cause the plunger to return beyond the rib.In this particular embodiment, the above mentioned coupling is of the male-female type.A substantially spherical head provided at a corresponding end of the plunger rod can engage in a corresponding mating hollow provided in the resilient material of the plunger.

While the above disclosed single use syringe has a comparatively simple construction,it has the great drawback that for making the mentioned rib there are necessary very complex and expensive molds,which negatively affects the cost of the finished syringe.

## SUMMARY OF THE INVENTION

The present invention sets out to provide a disposable syringe,or single use syringe,which can be constructed in a much simplier way and which,accordingly, is much more unexpensive than the above disclosed syringe.

In particular,the main object of the present invention is to provide a disposable syringe which is more unexpensive than known conventional syringes of the single use type.

This object is achieved by the syringe according to the present invention,which is characterized in that the inner surface of the cylindrical body in which the syringe plunger can slide is provided with a circular step,laying in a plane perpendicular to the axis of said cylindrical body,said circular step dividing the cylindrical body into two portions, the portion of the cylindrical body near the syringe needle having an inner diameter slightly larger than that of the remaining portion of the cylindrical body,said larger inner diameter portion having a sufficient length to receive said plunger;and in that the plunger comprises a material having such a resiliency as to provide tightness between the plunger and cylindrical body even as the plunger is arranged in the larger inner diameter portion of the cylindrical body.

As it will be apparent to those skilled in the plastic material molding art,the body of the syringe according to the present invention can be easily made in two hollow cylindrical portions of different diameters,which are then sealed to one another by a known sealing method,for example by ultrasounds,so as to provide the above mentioned step.

Moreover,in order to further reduce the cost of the syringe, the needle can be directly affixed to the larger diameter portion of the cylindrical body, and this can be performed by embedding the related end of the needle in said larger diameter portion of the cylindrical body as the latter is molded.


## BRIEF DESCRIPTION OF THE DRAWING


The invention will become mire apparent hereinafter from the following disclosure of a syringe according to the invention,with reference to the accompanying drawing, where:

Figure 1 is a longitudinal cross-sectional view of the syringe,with the syringe plunger being arranged in the position it assumes as the syringe is delivered;and

Figure 2 is a cross-sectional view like fig.1, with the syringe plunger arranged in its use position.


## DESCRIPTION OF THE PREFERRED EMBODIMENT


As is shown in the figures,the syringe 10 is provided with a hollow cylindrical body which essentially comprises two portions : a first portion 14 having a greater length and a smaller inner diameter,and a second portion 16 of a comparatively short length but sufficient to house a plunger 20 and having an inner diameter slightly greater than that of said first portion.The inner diameters of the mentioned portions 14 and 16 of the cylindrical body 12 will be so designed that, upon sealing said portions to one another,e.g by ultrasounds,a step 18 will be formed, as shown in the figures.

The second portion 16 of the cylindrical body 12 is provided with a tapering portion 22 of frustum of cone shape in which there is restrained a needle 24.

In the cylindrical body 12 a plunger 20 can tightly slide,said plunger being made of a known suitable resilient plastic material.As the plunger 20 is in its undeformed condition,it will obviously have a diameter greater than the inner diameter of the first portion 14 of the cylindrical body 12,in order to provide tightness between the plunger 20 and the inner surface of the first portion 14 of the cylindrical body 12.The plunger 20 can be driven by means of a plunger rod 26 one end 28 of which projects from the cylindrical body 12 and has a disc shape 28 in order to facilitate the gripping thereof.

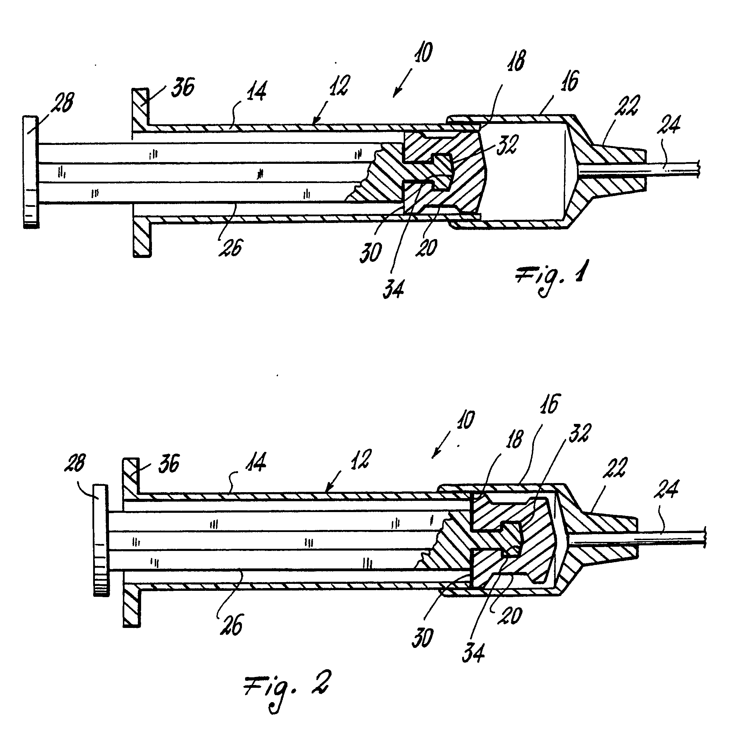

As is should be easily apparent,the syringe can be used in a very simple way.In fact it will be sufficient to conventionally suck the injectable liquid and then inject it in the usual manner by pushing,by means of the plunger rod 26, the plunger 20 to its end of stroke position.Thus the plunger will assume its position shown in figure 2.Since the material of the plunger 20 has a suitable resilience and since said plunger,after having passed the step 18,will be arranged in the portion 16 of the cylindrical body 12 having the greater diameter,said plunger will be slightly expanded so as to fit the greater diameter of this portion.The plunger,on the other hand,must still assure tightness between it and the inner surface of the portion 16,of greater inner diameter,of the cylindrical body 12.

If now,by driving the rod 26,one would attempt to cause the plunger 20 to move back,the peripheral edge of its rear portion 30 would abut against the step 18,thereby hindering this movement.

In order to prevent the plunger 20 from returning to the smaller diameter portion 14,by overcoming the step 18,because of forces sufficiently great to deform the plunger,a device can be provided, like that of the above mentioned Italian Patent Application N° 9,354 A/87,allowing the plunger rod to be separated from the plunger as on said plunger there is applied a pulling force greater than a set value,which value must however be less than that necessary for causing the plunger 20 to overcome,during its return movement,the step 18.

The drawing figures show a coupling between the plunger rod 26 and plunger 20 which has the above mentioned features.As stated,this coupling fails as the pulling force on the plunger rod 26 reaches a set value.The mushroom shaped end 32 of the plunger rod 26 is adapted to mate with an axial hollow 34 formed in the rear portion 30 of the plunger 20.As the pulling force on the plunger rod 26 becomes greater than the set value,the mushroom end 32 will project from its hollow 34, thereby the plunger 20 is prevented from exiting the greater inner diameter portion 16 of the cylindrical body 12.

In order to assure that the piston is held in the portion 14 of the cylindrical body,during the not use period,and prevent it from being accidentally brought beyond the step 18,which would render the syringe unusable,there are provided removable means (not shown in the figures for simplicity) adapted to prevent the plunger 20 from being push advanced. This can be obtained in a very simple way,for example by means of one or more manually breakable elements, extending from the fins 36 and operating as spacer members between said fins and the end disc 28 of the plunger rod 26.

Thus,the plunger is prevented from advancing unless said breakable elements are removed.

The disclosed syringe,owing to its particular characteristics,has a very reduced cost,which is even less than that of conventional disposable syringes.

## Claims

1- A medical use syringe,of the disposable type, characterized in that the inner surface of the cylindrical body in which the syringe plunger can slide is provided with a circular step,laying in a plane perpendicular to the axis of said cylindrical body,said circular step dividing the cylindrical body into two portions, the portion of the cylindrical body near the syringe needle having an inner diameter slightly larger than that of the remaining portion of the cylindrical body,said larger inner diameter portion having a sufficient length to receive said plunger;and in that the plunger compriges a material having such a resiliency as to provide tightness between the plunger and cylindrical body even as the plunger is arranged in the larger inner diameter portion of the cylindrical body.

2- A syringe according to claim 1,characterized in that the needle is directly coupled to the larger diameter portion of the cylindrical body.

3- A syringe according to claims 1 and 2,characterized in that between the plunger rod and the plunger there is provided a coupling device allowing the plunger rod to be separated from the plunger as on the plunger rod there is applied a pulling force greater than a set value,said set value being less than that necessary to cause the plunger to pass beyond said step during a return movement of said plunger.

4- A syringe according to claims 1 to3, characterized in that there are provided means for preventing said plunger from being accidentaly brought beyond said step,as said syringe is not used.

Fig. 1

Fig. 2

| | European Patent Office | |
|---|---|---|

## DOCUMENTS CONSIDERED TO BE RELEVANT

EP 90102473.7

| Category | Citation of document with indication, where appropriate, of relevant passages | Relevant to claim | CLASSIFICATION OF THE APPLICATION (Int Cl⁵) |
|---|---|---|---|
| P,X | <u>EP - A2 - 0 325 886</u> (CANDELA ESTRUCH) * Fig. 1,2,3,5; column 3, lines 10-25,31-33 * | 1-3 | A 61 M 5/50 A 61 M 5/315 |
| Y | | 4 | |
| Y | <u>US - A - 4 391 272</u> (J.STAEMPFLI) * Fig. 1a,6; column 2, lines 56-59; column 3, line 61 - column 4, line 6; column 7, lines 1-10 * | 4 | |
| A | | 2,3 | |
| P,A | <u>WO - A1 - 89/06 146</u> (J.VERLIER) * Fig. 7,8; page 4, paragraph 1; page 6, paragraph 3; page 9, paragraph 42 - page 10, paragraph 41 * | 1,2,4 | |

TECHNICAL FIELDS
SEARCHED (Int Cl⁵)

A 61 M 5/00

The present search report has been drawn up for all claims

| Place of search | Date of completion of the search | Examiner |
|---|---|---|
| VIENNA | 22-06-1990 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82